# EUROPEAN PATENT APPLICATION

(11) **EP 2 228 059 A1**
(43) Date of publication of application: **15.09.2010**
(21) Application number: 09155012.9
(22) Date of filing: 12.03.2009
(51) Int. Cl.: A61K 9/127, A61K 39/395

(54) **Chemotherapeutic composition for the treatment of cancer**

(71) Applicant: Universitätsspital Basel, 4031 Basel (CH)
(72) Inventor: The designation of the inventor has not yet been filed
(74) Representative: Vossius & Partner

(57) **Abstract**

The present invention is in the area of cancer treatment. In particular, the invention relates to chemotherapeutic compositions useful in the treatment of individuals suffering from cancer, particularly a cancer represented by a locally advanced or metastatic tumor. The present invention also provides the use of such composition as a pharmaceutical composition.

## Description

The present invention is in the area of cancer treatment. In particular, the invention relates to chemotherapeutic compositions useful in the treatment of individuals suffering from cancer, particularly a cancer represented by a locally advanced or metastatic tumor. The present invention also provides the use of such composition as a pharmaceutical composition.

Angiogenesis, the development of new blood vessels, is critical for tumor growth, invasion, and metastasis. Folkman (Folkman, 1971) observed that tumors require new vessel formation to grow beyond 1 to 2 mm³. In various solid tumors, microvessel density as a marker for angiogenesis is associated with recurrence, metastasis, and prognosis (Takebayashi et al., 1996).

Indeed, cell surface markers expressed by endothelial cells differ depending on the host organ, resulting in a tissue-specific zip code displayed on the endoluminal surface of the vessel. More importantly, endothelial cells lining new vessels that are being formed in a growing tumor express a different set of genes in comparison to those lining physiological vessels, which translates into a different expression of surface markers (Seaman et al., 2007, Cancer Cell, 11, 539-54).

Of the numerous growth factors involved in the complex process of angiogenesis, vascular endothelial growth factor (VEGF) is thought to be the most potent and specific stimulator of angiogenesis. The VEGF family comprises many isoforms, but VEGF-A (commonly known as VEGF) is implicated in angiogenesis. The effects of VEGF are mediated through two tyrosine kinase receptors based on endothelial cells, VEGF-1 (Flt-1) and VEGFR-2 (KDR/flk-1), with VEGFR-2 thought to provide the major contribution to angiogenesis (Millauer et al., 1996).

The VEGF-A / VEGFR2 axis is one of the major factors governing the angiogenic switch, which is the transition from the vascular phase of tumor development to the angiogenic phase and necessary to allow tumors to overcome growth limitations imposed by insufficient blood supply. Interestingly, the VEGFR2 is upregulated 3-5 fold on the epithelium of tumor-associated vessels in many cancer types (Plate et al., 1994, Int J Cancer, 59, 520-9). This upregulation probably is the consequence of a paracrine loop between the tumor cells producing VEGF-A and the endothelial cells (Shibuya, 1995, Adv Cancer Res, 67, 281-316). Not surprisingly, the activation of the VEGF-ANEGFR-2 axis and the differential expression of VEGFR-2 on tumor-associated endothelial cells make it a prime target for anti-angiogenic treatments in malignant tumors (reviewed by (Folkman, 2007, Nat Rev Drug Discov, 6, 273-86)). The first anti-angiogenic drug introduced into the clinic has been bevacizumab, a monoclonal antibody that traps VEGF-A, a VEGFR2-ligand. Clinical studies have proven the efficacy of bevacizumab in human breast, lung, colorectal and central nervous system cancers, thus confirming the concept of therapies directed against the tumor vasculature and, more generally speaking, the tumor stroma. Apart from targeting the growth factor itself, other experimental approaches aimed against tumor-driven neo-angiogenesis have included small molecule inhibitors or monoclonal antibodies suppressing the activity of the VEGF receptor (VEGF-R), thus blocking signaling through the VEGF/VEGFR-axis.

Therefore, VEGFR and its ligands have become attractive targets for cancer therapies in view of their pivotal role in angiogenesis, specificity, and association with outcome (for review, see (Hicklin & Ellis, 2005)). For example, bevacizumab (Avastin®), a MAb binding VEGF ligand, has demonstrated superior efficacy in patients with colorectal cancer when used in combination with standard chemotherapy compared to chemotherapy alone (Hurwitz et al., 2004).

Additional anti-VEGF strategies are currently under investigation, including small molecules affecting signaling or VEGF binding and antibodies targeting VEGF receptors, such as IMC-IC11 targeting VEGFR-2 (Posey et al., 2003). Its murine counterpart, namely DC101, has demonstrated clear activity and proof-of-concept in several preclinical in vitro and in vivo models (Shaheen et al., 2001; Vosseler et al., 2005). Another interesting antigen for targeting angiogenesis is vascular endothelial (VE)-cadherin, also known as CD144 and as an adhesion molecule uniquely expressed in endothelial cells (Martin et al., 2005).

The distribution of most current drugs in the body is determined by their physicochemical properties. This constitutes a major obstacle to the development of successful therapies, since drug delivery is not limited to the diseased organ but also to the whole body, resulting in significant side-effects and a comparatively low concentration of the drug in the target tissue. Certain nanoparticles, such as sterically stabilized liposomes, have already been optimized for prolonged circulation and redirection of drug, which can yield superior accumulation in tumors via a process often referred to as the "enhanced permeability and retention" (EPR) effect (for review, Maeda H. et al., Eur J Pharm Biopharm. 2009 Mar;71(3):409-19).

Huge efforts have been made in the past to identify specific cell surface markers on tumor cells and to direct anti-cancer agents selectively to malignant cells. Immunoliposomes (ILs), in which monoclonal antibody (MAb) fragments are conjugated to liposomes, represent the next generation of molecularly targeted drug delivery systems. By combining the tumor targeting properties of MAbs with the pharmacokinetic and drug delivery advantages of liposomes, immunoliposomes offer the promise of selective drug delivery to tumor cells, including internalization and intracellular drug release within targeted cells (Noble C. et al., Expert Opin Ther Targets. 2004 Aug; 8(4):335-53). Using this modular approach, immunoliposomes have been developed that target epidermal growth factor receptor (EGFR, ErbB1) successfully (Mamot C. et al., Cancer Research 2003 and 2005).

Immunoliposomal drug delivery is designed to mediate specific and efficient transport of drugs to target cells. Now, evidence is emerging that liposomes and in particular ILs might also be able to bypass drug resistance mechanisms, which continue to be a major challenge in cancer treatment.

Intrinsic or acquired drug resistance occurs frequently in most cancers, and often involves resistance to multiple agents simultaneously (multidrug resistance, MDR). A number of mechanisms for drug resistance have been described, including overexpressed drug export pumps, such as P-glycoprotein (PGP) and multidrug-resistance protein (MRP); decreased drug uptake, such as altered folate carriers; inactivation of drugs, such as via glutathione-mediated reduction; overexpression of target enzymes, such as upregulated thymidylate synthase; altered drug targets, such as topoisomerase Il; increased DNA repair capacity; reduced ability to undergo apoptosis; and others (reviewed in (Broxterman et al., 2003)). Specific inhibitors of these resistance mechanisms have been widely pursued as a means to restore drug sensitivity (for review, see (Leonard et al., 2002)).

However, progress towards therapeutic success has been hampered by such issues as inadequate specificity, both predictable and unforeseen toxicities, uncertainly about the true prevalence and contribution of the known resistance mechanisms, paucity of predictive assays to identify tumors dependent upon particular mechanisms, and multiplicity and redundancy of resistance mechanisms.

The rationale for overcoming drug resistance by immunoliposomal drug delivery systems is based on achieving selective drug accumulation in tumor tissues, tumor cells, or even compartments of tumor cells. Remarkably, even long circulating non-targeted liposomes can increase drug deposition in solid tumors, which may help overcoming drug resistance. Ligand-targeted liposomes, such as ILs, which can internalize in tumor cells for intracellular drug delivery and maximal drug efficacy might modify controlled release of their content and increase drug bioavailability (Mamot et al., 2003b; Noble et al., 2004).

It was now shown within the scope of the present invention that the approach applied in EGFR-targeting can not only be used against antigen-presenting tumor cells themselves but, alternatively, also against the tumor stroma, including the tumor-associated vasculature, which offers some potential advantages.

While it is true that drug resistance mechanisms may be overcome by immunoliposomal drug delivery systems as shown for ILs targeting EGFR tumor cell-associated antigens, there is still a need for the provision of a chemotherapeutic composition, which is capable of escaping the development of resistance mechanisms, such as, for example, a composition, which enables the targeting of tumor-associated endothelial cells which are not expected to develop such resistance mechanisms, particularly a therapeutic composition, which is based on a drug delivery system comprising VEGFR-targeted ILs.

The gist of the present invention therefore lies in the provision of a chemotherapeutic composition useful in the treatment of cancer. Moreover, it was now surprisingly found within the scope of the present invention that the use of this composition enables targeting of tumor-associated endothelial cells, which are not expected to develop resistance mechanisms similar to tumor cells. Furthermore, tumor cells exhibiting VEGFR antigens can also be targeted by using the composition of the present invention.

The technical problem underlying the present invention is the provision of a chemotherapeutic composition useful in the treatment of cancer.

The technical problem is solved by the provision of the embodiments characterized in the claims.

The present invention relates in a first embodiment to the provision of (1) an immunoliposome comprising an antibody or an antibody fragment, which recognizes and binds to an VEGF receptor (VEGFR) antigen on the surface of a tumor cell or a tumor-asscociated endothelial cell and further encapsulating in the liposome an anti-tumor compound, for treatment of cancer, particularly a cancer represented by a locally advanced or metastatic tumor, in an individual, particularly animal or human.

In a second embodiment, (2) an immunoliposome according to embodiment (1) is provided, wherein the VEGFR antigen is selected from a group consisting of VEGFR1, VEGFR2 or VEGFR3 antigens or combinations thereof.

In a third embodiment of the present invention, (3) an immunoliposome according to embodiment (2) is provided, wherein the VEGFR antigen is a VEGFR2 antigen.

In another embodiment as described herein, (4) an immunoliposome according to embodiments (1) to (3) is provided, wherein said endothelial cells are cells lining new vessels that are being formed in a growing tumor.

In a further embodiment, (5) an immunoliposome according to any of the preceding embodiments is provided, wherein the liposome encapsulates an anti-tumor compound, particularly a cytostatic compound.

In a further embodiment, (6) an immunotiposome according to any of the preceding embodiments is provided, wherein the anti-tumor compound is a compound selected from the group consisting of daunomycin, idarubicin, mitoxantrone, mitomycin, cisplatin and other Platinum analogs, vincristine, epirubicin, aclacinomycin, methotrexate, etoposide, doxorubicin, cytosine arabinoside, fluorouracil and other fluorinated pyrimidines, purines, or nucleosides, especially gemcitabine, bleomycin, mitomycin, plicamycin, dactinomycin, cyclophosphamide and derivatives thereof, thiotepa, BCNU, paclitaxel, vinorelbine, docetaxel and other taxane derivatives and isolates, camptothecins, polypeptides, a nucleic acid, a nucleic acid having a phosphorothioate internucleotide linkage, and a nucleic acid having a polyamide internucleotide linkage.

In a particular embodiment, (7) an immunoliposome according to embodiment (6) is provided, wherein the cytotoxic compound is a compound selected from the group consisting of doxorubicin, epirubicin and vinorelbine.

In one embodiment, (8) an immunoliposome according to any of the preceding embodiments is provided, wherein the tumor is still progressing.

In a further embodiment, an immunoliposome according to any of the preceding embodiments is provided, wherein an individual, particularly animal or human, has developed a multi-drug resistance.

In another embodiment, (9) an immunoliposome according to any of the preceding embodiments is provided, wherein the treatment leads to a regression of the disease.

In a further embodiment, (10) an immunoliposome according to any of the preceding embodiments is provided, wherein the treatment shows no or substantially no toxic side effects.

In a further embodiment, (11) an immunoliposome according to any of the preceding embodiments is provided, wherein the treatment does not show substantial toxicity.

In one embodiment, (12) an immunoliposome according to any of the preceding embodiments is provided, wherein the treatment shows no or substantially no toxic side effects at a concentration of between 5 mg/m² and 80 mg/m2.

In a further embodiment, (13) an immunoliposome according to any of the preceding embodiments is provided, wherein the treatment shows no or substantially no toxic side effects at a concentration of up to 40 mg/m2.

In another embodiment, (14) an immunoliposome according to any of the preceding embodiments is provided, wherein the antibody or antibody fragment is covalently bound to the liposome membrane.

In one embodiment, (15) an immunoliposome according to any of the preceding embodiments is provided, wherein the antibody is covalently conjugated to the terminus of a linker molecule anchored to the liposome.

In another embodiment, (16) an immunoliposome according to the previous embodiment is provided, wherein the linker molecule is a hydrophilic polymer, particularly a polyethylene glycol.

In a further embodiment, (17) an immunoliposome according to any of the preceding embodiments is provided, wherein the antibody is a monoclonal antibody directed to the ligand-binding extracellular domain of the VEGF receptor, particularly the ligand-blinding extracellular domain of the VEGF-2 receptor, particularly a monoclonal antibody such as, for example, DC101.

In a further embodiment, (18) an immunoliposome according to any of the preceding embodiments is provided for the treatment of a cancer in an individual, particularly animal or human, selected from the group consisting of Kaposi's sarcoma, recurrent ovarian cancer, breast cancer, soft tissue sarcoma, glioma, melanoma, mesothelioma, transitional cell carcinoma of the urothelial tract, endometrial, pancreatic, small-cell and non-small-cell lung, hepatocellular, renal cell, esophageal, colorectal, anal, vaginal, vulvar, prostate, basal cell carcinoma of the skin head and neck, and cholangio carcinoma, which cancer is particularly represented by a locally advanced or metastatic tumor, particularly a tumor comprising tumor cells exhibiting VEGF receptor (VEGFR) antigen on the cell surface or, particularly, a tumor comprising tumor-asscociated endothelial cells, but especially endothelial cells lining new vessels that are being formed in a growing tumor and exhibiting VEGF receptor (VEGFR) antigen on the cell surface.

In a further embodiment, (19) an immunoliposome according to any of the preceding embodiments is provided for the treatment of a cancer in an individual, particularly animal or human, selected from the group consisting of prostate, pancreatic, kidney, urothelial, oesophageal, head and neck, colonrectal, a hepatocellular cancer and a mesothelioma, which cancer is particularly represented by locally advanced or metastatic tumor, particularly a tumor comprising tumor cells exhibiting VEGF receptor (VEGFR) antigen on the cell surface or, particularly, a tumor comprising tumor-asscociated endothelial cells, but especially endothelial cells lining new vessels that are being formed in a growing tumor and exhibiting VEGF receptor (VEGFR) antigen on the cell surface.

In another embodiment, (20) an immunoliposome according to any of the preceding embodiments is provided for use in the treatment of an individual, particularly animal or human, who has cancer, particularly a cancer represented by a locally advanced or metastatic tumor, particularly a tumor comprising tumor cells exhibiting VEGF receptor (VEGFR) antigen on the cell surface or, particularly, a tumor comprising tumor-asscociated endothelial cells, but especially endothelial cells lining new vessels that are being formed in a growing tumor and exhibiting VEGF receptor (VEGFR) antigen on the cell surface.

In one embodiment, the present invention relates to the (21) use of an immunoliposome according to any of the preceding embodiments for the preparation of a pharmaceutical composition for the treatment of cancer, particularly a cancer represented by a locally advanced or metastatic tumor, particularly a tumor comprising tumor cells exhibiting VEGF receptor (VEGFR) antigen on the cell surface or, particularly, a tumor comprising tumor-asscociated endothelial cells, but especially endothelial cells lining new vessels that are being formed in a growing tumor and exhibiting VEGF receptor (VEGFR) antigen on the cell surface

In a further embodiment, the present invention relates to (22) a method of using an immunoliposome or a pharmaceutical composition according to any of the preceding embodiments for the preparation of a pharmaceutical for use in the treatment of an individual, particularly animal or human, who has cancer, particularly a cancer represented by a locally advanced or metastatic tumor, particularly a tumor comprising tumor cells exhibiting VEGF receptor (VEGFR) antigen on the cell surface or, particularly, a tumor comprising tumor-asscociated endothelial cells, but especially endothelial cells lining new vessels that are being formed in a growing tumor and exhibiting VEGF receptor (VEGFR) antigen on the cell surface

In another embodiment, (23) a pharmaceutical composition comprising an immunoliposome according to any of the preceding embodiments is provided, together with a pharmaceutically acceptable carrier or excipient or a diluent for the treatment of cancer represented by a locally advanced or metastatic tumor, particularly a tumor comprising tumor cells exhibiting VEGF receptor (VEGFR) antigen on the cell surface or, particularly, a tumor comprising tumor-asscociated endothelial cells, but especially endothelial cells lining new vessels that are being formed in a growing tumor and exhibiting VEGF receptor (VEGFR) antigen on the cell surface in an individual, particularly animal or human.

In a particular embodiment, (24) a pharmaceutical composition according to embodiment (23) is provided, for the treatment of cancer comprising ovarian, cervical, endometrial, gastric, bladder, a uterine sarcoma, a multiple myeloma, and soft tissue and bone sarcomas, particularly breast cancer.

In a particular embodiment, (25) a pharmaceutical composition according to embodiment (23) is provided, for the treatment of a colorectal cancer.

In one embodiment, the present invention also relates to (26) a pharmaceutical composition comprising an immunoliposome according to any of the preceding claims, wherein said immunoliposome encapsulates doxorubicin and further comprises antibody DC101 MAb or a fragment thereof, which still exhibits the specific binding properties of said antibody.

In particular, the present invention relates to (27) a pharmaceutical composition according to embodiments (23) to (26) for use in the treatment of an individual, particularly animal or human, who has cancer, particularly a cancer represented by a locally advanced or metastatic tumor, particularly a tumor comprising tumor cells exhibiting VEGF receptor (VEGFR) antigen on the cell surface or, particularly, a tumor comprising tumor-asscociated endothelial cells, but especially endothelial cells lining new vessels that are being formed in a growing tumor and exhibiting VEGF receptor (VEGFR) antigen on the cell surface.

In one embodiment, the present invention relates to the (28) use of the pharmaceutical composition according to any of the preceding embodiments for the preparation of a pharmaceutical composition for the treatment of cancer, particularly a cancer represented by a locally advanced or metastatic tumor, particularly a VEGFR-positive tumor.

In another embodiment of the present invention, (29) a method of treatment of an individual, particolarly animal or human is contemplated, who has cancer, particularly a cancer represented by a locally advanced or metastatic tumor, particularly a tumor comprising tumor cells exhibiting VEGF receptor (VEGFR) antigen on the cell surface or, particularly, a tumor comprising tumor-asscociated endothelial cells, but especially endothelial cells lining new vessels that are being formed in a growing tumor and exhibiting VEGF receptor (VEGFR) antigen on the cell surface, wherein (a) the immunoliposomes or the pharmaceutical composition of any of the preceding claims is administered to an individual, particularly animal or human; and (b) the effect of the treatment is monitored based on one or more parameters selected from the group consisting of tumor volume, tumor growth, tumor cell proliferation, number of apoptotic cells and microvessel density.

In a further embodiment of the invention, (30) a method for disruption of small blood vessels comprising tumor cells or tumor-associated endothelial cells is contemplated, wherein (a) the immunoliposome or the pharmaceutical composition of any of the preceding claims is administered to an individual, particularly animal or human; and (b) the disruption of said blood vessels is monitored by immunofluorescence staining of the target cells.

In another embodiment, an immunoliposome according to the invention and as described herein before is provided for treatment of an individual (animal or human) belonging to the group of non-responders, who has cancer, particularly a cancer represented by a locally advanced or metastatic tumor as described herein before, particularly to a patient who has developed a multi drug resistance, wherein said immunoliposome has an IC50, determined in a standard MTT assay, of between 1.0 µg /ml and 5.0 µg /ml, particularly of between 0.8 µg /ml and 3.5 µg /ml, particularly of between 0.7 µg /ml and 2.5 µg /ml, particularly of between 0.6 µg /ml and 2.0 µg /ml, particularly of between 0.5 µg /ml and 1.5 µg /ml, particularly of between 0.4 µg /ml and 1.0 µg /ml, particularly of between 0.3 µg /ml and 0.5 µg /ml, particularly of between 0.2 µg /ml and 0.4 µg /ml. The immunoliposome is particularly an immunoliposome comprising doxorubicin.

In one embodiment of the invention the anti-VEGFR immunoliposome is given at a dose level of 10 mg/m² and 40 mg/m² body surface, particularly between 30 mg/m² and 50 mg/m², particularly between 40 mg/m² and 60 mg/m², particularly between 50 mg/m² and 70 mg/m², particularly between 60 mg/m² and 80 mg/m², particularly between 70 mg/m² and 90 mg/m², particularly between 75 mg/m² and 100 mg/ m², given as a short infusion every 2 to 6 weeks, particularly every 3 to 5 weeks, but especially every 4 weeks. By a short infusion an infusion time of at least 10 min, particularly of at least 20 min, particularly of at least 30 min, particularly of at least 40 min, particularly of at least 60 min, particularly of at least 90 min, particularly of at least 120 min, particularly of at least 240 min is meant.

In one embodiment, the invention relates to an immunoliposome as described herein before, particularly an immunoliposome comprising a doxorubicin compound, for treatment of an individual (animal or human) who is suffering from cancer, particularly a cancer represented by a locally advanced or metastatic tumor, for example glioblastoma, particularly a tumor comprising tumor cells exhibiting VEGF receptor (VEGFR) antigen on the cell surface or, particularly, a tumor comprising tumor-asscociated endothelial cells, but especially endothelial cells lining new vessels that are being formed in a growing tumor and exhibiting VEGF receptor (VEGFR) antigen on the cell surface, wherein the treatment shows no or substantially no toxic side effects at an immunoliposome concentration of between 5 mg/m² and 100 mg/m² of body surface, particularly between 10 mg/m² and 90 mg/m², particularly between 20 mg/m² and 80 mg/m², particularly between 30 mg/m² and 70 mg/m², particularly between 40 mg/m² and 60 mg/m², particularly between 60 mg/m² and 80 mg/m², particularly between 70 mg/m² and 90 mg/m², particularly between 75 mg/m2 and 100 mg/ m2.

In one embodiment, an immunoliposome according to the invention and as described herein before is provided for treatment of a human patient in a clinical set-up, wherein said patient is suffering from a cancer selected from the group consisting of Kaposi's sarcoma, recurrent ovarian cancer, soft tissue sarcoma, glioma, melanoma, mesothelioma, transitional cell carcinoma of the urothelial tract, endometrial, pancreatic, small-cell and non-small-cell lung, hepatocellular, renal cell, esophageal, colorectal, anal, vaginal, vulvar, prostate, basal cell carcinoma of the skin head and neck, and cholangio carcinoma, which cancer is particularly represented by a locally advanced or metastatic tumor, particularly a tumor comprising tumor cells exhibiting VEGF receptor (VEGFR) antigen on the cell surface or, particularly, a tumor comprising tumor-asscociated endothelial cells, but especially endothelial cells lining new vessels that are being formed in a growing tumor and exhibiting VEGF receptor (VEGFR) antigen on the cell surface.

In one embodiment, an immunoliposome is provided according to the invention and as described herein before, or a pharmaceutical composition comprising said immunoliposome, for treatment of a human patient in a clinical set-up, wherein said patient is suffering from a cancer selected from the group consisting of prostate, pancreatic, kidney, oesophageal, colon, and rectal cancer, which cancer is particularly represented by locally advanced or metastatic tumor, particularly a tumor comprising tumor cells exhibiting VEGF receptor (VEGFR) antigen on the cell surface or, particularly, a tumor comprising tumor-asscociated endothelial cells, but especially endothelial cells lining new vessels that are being formed in a growing tumor and exhibiting VEGF receptor (VEGFR2) antigen on the cell surface.

In one embodiment of the invention, an immunoliposome is provided for treatment of an individual, particularly animal or human, who has cancer, particularly a cancer represented by a locally advanced or metastatic tumor, particularly particularly a tumor comprising tumor cells exhibiting VEGF receptor (VEGFR) antigen on the cell surface or, particularly, a tumor comprising tumor-asscociated endothelial cells, but especially endothelial cells lining new vessels that are being formed in a growing tumor and exhibiting VEGF receptor (VEGFR) antigen on the cell surface as described herein before, wherein a response rate is achieved of between 5% and 10%, particularly of between 7% and 15%, particularly of between 9% and 20%, particularly between 12% and 25%, particularly between 18% and 30%, particularly between 22% and 35%, particularly between 28% and 40%, particularly between 32% and 45%, particularly between 38% and 50%, particularly between 42% and 55%, particularly between 48% and 60%, particularly between 52% and 60%, particularly between 52% and 70%, particularly between 52% and 75%, particularly between 58% and 80%, particularly between 62% and 85%, particularly between 68% and 90%, particularly between 72% and 95%, and up to 100%.

### Definitions

The term "immunoliposomes" as used herein refers to targeted liposomes having targeting ligands or affinity moieties attached to the surface of the liposomes. The targeting ligands may be antibodies or fragments thereof, in which case the liposomes are referred to as "immunoliposomes". When administered systemically, targeted liposomes deliver the entrapped therapeutic agent to a target tissue, region or cell. Because targeted liposomes are directed to a specific region or cell, healthy tissue is not exposed to the therapeutic agent. Such targeting ligands can be attached directly to the liposomes' surfaces by covalent coupling of the targeting ligand to the polar head group residues of liposomal lipid components (see, for example, U.S. Patent No. 5,013,556).

The term "liposome" refers to a small, spherical vesicle composed of lipids, particularly vesicle-forming lipids capable of spontaneously arranging into lipid bilayer structures in water with its hydrophobic moiety in contact with the interior, hydrophobic region of the bilayer membrane, and its head group moiety oriented toward the exterior, polar surface of the membrane. Vesicle-forming lipids have typically two hydrocarbon chains, particularly acyl chains, and a head group, either polar or nonpolar. Vesicle-forming lipids are either composed of naturally-occurring lipids or of synthetic origin, including the phospholipids, such as phosphatidylcholine, phosphatidylethanolamine, phosphatidic acid, phosphatidylinositol, and sphingomyelin, where the two hydrocarbon chains are typically between about 14-22 carbon atoms in length, and have varying degrees of unsaturation. The above-described lipids and phospholipids whose acyl chains have varying degrees of saturation can be obtained commercially or prepared according to published methods. Other suitable lipids for use in the composition of the present invention include glycolipids and sterols such as cholesterol and its various analogs which can also be used in the liposomes.

Cationic lipids, which typically have a lipophilic moiety, such as a sterol, an acyl or diacyl chain, and where the lipid has an overall net positive charge can also be suitably used in liposomes. The head group of the lipid typically carries the positive charge. Exemplary cationic lipids include 1,2-dioleyloxy-3-(trimethylamino) propane (DOTAP); N-[1-(2,3,-ditetradecyloxy)propyl]-N,N-dimethyl-N-hydroxyethylammonium bromide (DMRIE); N-[1-(2,3,-dioleyloxy)propyl]-N,N-dimethyl-N-hydroxy ethylammonium bromide (DORIE); N-[1-(2,3-dioleyloxy) propyl]-N,N,N-trimethylammonium chloride (DOTMA); 3 [N-(N',N'-dimethylaminoethane) carbamoly]cholesterol (DC-Chol); and dimethyldioctadecylammonium (DDAB). The cationic vesicle-forming lipid may also be a neutral lipid, such as dioleoylphosphatidyl ethanolamine (DOPE) or an amphipathic lipid, such as a phospholipid, derivatized with a cationic lipid, such as polylysine or other polyamine lipids.

The liposomes can include a vesicle-forming lipid derivatized with a hydrophilic polymer to form a surface coating of hydrophilic polymer chains on the liposomes surface. A vesicle-forming lipid, in particular a phospholipid, such as distearoyl phosphatidylethanolamine (DSPE), may be covalently attached to a hydrophilic polymer, which forms a surface coating of hydrophilic polymer chains around the liposome. Hydrophilic polymers suitable for derivatization with a vesicle-forming lipid include polyvinylpyrrolidone, polyvinylmethylether, polymethyloxazoline, polyethyloxazoline, polyhydroxypropyloxazoline, polyhydroxypropylmethacrylamide, polymethacrylamide, polydimethylacrylamide, polyhydroxypropylmethacrylate, polyhydroxyethylacrylate, hydroxymethylcellulose, hydroxyethylcellulose, polyethyleneglycol, polyaspartamide and hydrophilic peptide sequences. The polymers may be employed as homopolymers or as block or random copolymers.

A preferred hydrophilic polymer chain is polyethyleneglycol (PEG), preferably as a PEG chain having a molecular weight between 200-20,000 daltons, more preferably between 500-10,000 daltons, still more preferably between 750-5000 daltons. Methoxy or ethoxy-capped analogs of PEG are also preferred hydrophilic polymers, commercially available in a variety of polymer sizes, e.g., 120-20,000 Daltons.

Additional polymer chains added to the lipid mixture at the time of Liposome formation and in the form of a lipid-polymer conjugate result in polymer chains extending from both the inner and outer surfaces of the liposomal lipid bilayers. Addition of a lipid-polymer conjugate at the time of liposome formation is typically achieved by including between 0.5-20 mole percent of the polymer-derivatized lipid with the remaining liposome forming components, e.g., vesicle-forming lipids.

Preparation of vesicle-forming lipids derivatized with hydrophilic polymers has been described, for example in U.S. Pat. No. 5,395,619, in U.S. Pat. No. 5,013,556, in U.S. Pat. No. 5,631,018 and in WO 98/07409. It will be appreciated that the hydrophilic polymer may be stably coupled to the lipid, or coupled through an unstable linkage, which allows the coated liposomes to shed the coating of polymer chains as they circulate in the bloodstream or in response to a stimulus.

The term "immunoliposome dosage" or "immunoliposome concentration" generally refers to the concentration of the anti-cancer agent entrapped in the liposome.

The term "comprise" is generally used in the sense of include, that is to say permitting the presence of one or more features or components.

As used in the specification and claims, the singular form "a", "an" and "the" include plural references unless the context clearly dictates otherwise. For example, the term "a patient" includes a plurality of patients.

The term "VEGF Receptor" or "VEGFR" is an art recognized term and used herein synonymously and is understood to refer to a receptor protein which is a member of the class V family of Receptor Tyrosine Kinases (RTKs), which includes VEGFR. The VEGF ligands bind to and activate three structurally similar type III receptor tyrosine kinases, designated VEGFR1 (also known as FLT1), VEGFR2 (also known as KDR) and VEGFR3 (also known as FLT4) (Ellis & Hicklin, Nature Reviews Cancer, 8, 579-591, 2008). As a target antigen, VEGFR is a readily accessible cell surface receptor, which is overexpressed in many solid tumors.

The term "VEGFR-positive tumor" as used herein is understood to refer to a tumor that contains at least 1%, particularly at least 2%, 3%, 4% or 5%, particularly at least 10%, VEGFR positive cells, detected e.g. by an immunohistochemistry test such as, for example the ABC-kit of Vectastain (Vector Laboratories), Sigma Fast (Sigma) or AEC-kit (Vector Laboratories).

The phrase "pharmaceutically acceptable" refers to molecular entities and compositions that are physiologically tolerable and do not typically produce an allergic or similar untoward reaction, such as gastric upset, dizziness and the like, when administered to am individual comprising human and/or animal.

The terms "antibody" or "antibodies" as used herein is an art-recognized term and is understood to refer to molecules or active fragments of molecules that bind to known antigens, particularly the terms "antibody" or "antibodies" refer to immunoglobulin molecules and to immunologically active portions of immunoglobulin molecules, i.e molecules that contain a binding site that immunospecifically binds an antigen. The immunoglobulin according to the invention can be of any type (IgG, IgM, IgD, IgE, IgA and IgY) or class (IgG1, IgG2, IgG3, IgG4, IgA1 and IgA2) or subclasses of immunoglobulin molecule.

"Antibodies" are intended within the scope of the present invention to include monoclonal antibodies, polyclonal, chimeric, single chain, bispecific, simianized, human and humanized antibodies as well as active fragments thereof. The term "fragment" refers to a part or portion of an antibody or antibody chain comprising fewer amino acid residues than an intact or complete antibody or antibody chain. Examples of active fragments of molecules that bind to known antigens include separated light and heavy chains, Fab, Fab/c, Fv, Fab', and F(ab')₂ fragments, including the products of an Fab immunoglobulin expression library and epitope-binding fragments of any of the antibodies and fragments mentioned above. Fragments can be obtained via chemical or enzymatic treatment of an intact or complete antibody or antibody chain, Fragments can also be obtained by recombinant means. Exemplary fragments include Fab, Fab', F(ab')₂, Fabc and/or Fv fragments. The term "antigen-binding fragment" refers to a polypeptide fragment of an immunoglobulin or antibody that binds antigen or competes with intact antibody (*i*.*e*., with the intact antibody from which they were derived) for antigen binding (*i*.*e*., specific binding).

Antibody-binding fragments are produced by recombinant DNA techniques, or by enzymatic or chemical cleavage of intact immunoglobulins. Binding fragments include Fab, Fab', F(ab')₂, Fabc, Fv, single chains, and single-chain antibodies.

These active fragments can be derived from a given antibody by a number of techniques. For example, purified monoclonal antibodies can be cleaved with an enzyme, such as pepsin, and subjected to HPLC gel filtration. The appropriate fraction containing Fab fragments can then be collected and concentrated by membrane filtration and the like.

Monoclonal antibodies are also envisaged in the present invention. For the preparation of monoclonal antibodies, any technique which provides antibodies produced by continuous cell line cultures can be used. Examples for such techniques includes the hybridoma technique, the trioma technique, the human B-cell hybridoma technique and the EBV-hybridoma technique to produce human monoclonal antibodies (Shepherd and Dean (2000), Monoclonal Antibodies: A Practical Approach, Oxford University Press, Goding and Goding (1996), Monoclonal Antibodies: Principles and Practice - Production and Application of Monoclonal Antibodies in Cell Biology, Biochemistry and immunology, Academic Pr Inc, USA).

The antibody derivatives can also be produced by peptidomimetics. Further, techniques described for the production of single chain antibodies (see, inter alia, US Patent 4,946,778) can be adapted to produce single chain antibodies specifically recognizing the polypeptide of the invention. Also, transgenic animals may be used to express humanized antibodies to the polypeptide of the invention.

The terms "specifically binds" or "specific binding properties", as used herein, refer to a binding reaction that is directed to the ligand-binding extracellular domain of the VEGF receptor and an antibody or fragments thereof in the presence of a heterogeneous population of proteins and other biologies. Thus, under designated assay conditions, the specified antibodies and polypeptides bind to one another and do not bind in a significant amount to other components present in a sample. Specific binding to a target analyze under such conditions may require a binding moiety that is selected for its specificity for a particular target analyte. A variety of immunoassay formats may be used to select antibodies specifically reactive with a particular antigen. For example, solid-phase ELISA immunoassays are routinely used to select monoclonal antibodies specifically immunoreactive with an analyte. See Shepherd and Dean (2000), Monoclonal Antibodies: A Practical Approach, Oxford University Press and/ or Howard and Bethell (2000) Basic Methods in Antibody Production and Characterization, Crc. Pr. Inc. for a description of immunoassay formats and conditions that can be used to determine specific immunoreactivity. Typically a specific or selective reaction will be at least twice background signal to noise and more typically more than 10 to 100 times greater than background.

A "chimeric antibody" is an antibody in which one or more regions of the antibody are from one species of animal and one or more regions of the antibody are from a different species of animal. A preferred chimeric antibody is one which includes regions from a primate immunoglobulin. A chimeric antibody for human clinical use is typically understood to have variable regions from a non-human animal, e.g. a rodent, with the constant regions from a human. In contrast, a humanized antibody uses CDRs from the non-human antibody with most or all of the variable framework regions from and all the constant regions from a human immunoglobulin. A human chimeric antibody is typically understood to have the variable regions from a rodent. A typical human chimeric antibody has human heavy constant regions and human light chain constant regions with the variable regions of both the heavy and light coming from a rodent antibody. A chimeric antibody may include some changes to a native amino acid sequence of the human constant regions and the native rodent variable region sequence. Chimeric and humanized antibodies may be prepared by methods well known in the art including CDR grafting approaches (see, e.g., U.S. Patent Nos. 5,843,708; 6,180,370; 5,693,762; 5,585,089; 5,530,101), chain shuffling strategies (see e.g., U.S. Patent No. 5,565,332; (16), molecular modelling strategies (U.S. Patent No. 5,639,641), and the like.

A "humanized antibody" refers to a type of engineered antibody which incorporates at least one humanized immunoglobulin or antibody chain or fragment thereof, particularly at least one humanized light or heavy chain. Said humanized immunoglobulin or antibody chain or fragment thereof, but particularly the at least one humanized light or heavy chain is derived from a non-human source, particularly a non-human antibody, typically of rodent origin. Said non-human contribution to the humanized antibody is typically provided in form of at least one CDR region which is interspersed among framework regions derived from one (or more) human immunoglobulin(s). In addition, framework support residues may be altered to preserve binding affinity.

The humanized antibody may further comprise constant regions (*e*.*g*., at least one constant region or portion thereof, in the case of a light chain, and preferably three constant regions in the case of a heavy chain).

Methods to obtain "humanized antibodies" are well known to those skilled in the art. (Queen et al., Proc. Natl Acad Sci, USA, 86: 10029-10032 (1989); Hodgson et al., Bio/Technology, 9:421 (1991)).

A "humanized antibody" may also be obtained by a novel genetic engineering approach that enables production of affinity-matured human-like polyclonal antibodies in large animals such as, for example, rabbits (http://www.rctech.com/bioventures/- therapeutic.php).

An "internalizing antibody" is an antibody that, upon binding to a receptor or other ligand on a cell surface, is transported into the cell, for example, into a lysozyme or other organelle or into the cytoplasm.

The immunoliposome composition of the invention thus also includes an antibody or antibody fragment including Fab, Fab', F(ab')₂, Fabc, Fv, single chains, and single-chain antibodies that specifically recognizes and bind to EGF receptor on the surface of a tumor derived cell. In another embodiment, the antibody comprises at least one binding domain which specifically binds the EGR receptor on the surface of a tumor-derived cell. In an alternate embodiment, the antibody is a single chain antibody comprising at least one binding domain which specifically binds EGF receptor on the surface of a tumor-derived cell.

Antibodies may be attached to a liposome by covalent methods known in the art. For attaching an antibody covalently to a liposome, a derivatized lipid containing an end-functionalized polyethylene glycol chain is incorporated into liposomes. After liposome formation, the end-functionalized group can react with an antibody for antibody coupling to a liposome.

There are a wide variety of techniques for attaching a selected hydrophilic polymer to a selected lipid and activating the free, unattached end of the polymer for reaction with a selected ligand, and in particular, the hydrophilic polymer polyethyleneglycol (PEG) has been widely studied (18; 19; 20; 21; 22).

Generally, the PEG chains are functionalized to contain reactive groups suitable for coupling with, for example, sulfhydryls, amino groups, and aldehydes or ketones (typically derived from mild oxidation of carbohydrate portions of an antibody) present in a wide variety of ligands. Examples of such PEG-terminal reactive groups include maleimide (for reaction with sulfhydryl groups), N-hydroxysuccinimide (NHS) or NHS-carbonate ester (for reaction with primary amines), hydrazide or hydrazine (for reaction with aldehydes or ketones), iodoacetyl (preferentially reactive with sulfhydryl groups) and dithiopyridine (thiol-reactive). Liposomes carrying an entrapped agent and bearing surface-bound targeting ligands, i.e., targeted, therapeutic liposomes, are prepared by any of these approaches. A preferred method of preparation is the insertion method, where preformed liposomes and are incubated with the targeting conjugate to achieve insertion of the targeting conjugate into the liposomal bilayers. In this approach, liposomes are prepared by a variety of techniques, such as those detailed in (23), and specific examples of liposome prepared in support of the present invention will be described below. Typically, the liposomes are multilamellar vesicles (MLVs), which can be formed by simple lipid-film hydration techniques. In this procedure, a mixture of liposome-forming lipids of the type detailed above dissolved in a suitable organic solvent is evaporated in a vessel to form a thin film, which is then covered by an aqueous medium. The lipid film hydrates to form MLVs, typically with sizes between about 0.1 to 10 microns. The liposomes can include a vesicle-forming lipid derivatized with a hydrophilic polymer to form a surface coating of hydrophilic polymer chains on the liposomes surface. Addition of a lipid-polymer conjugate is optional, since after the insertion step, described below, the liposomes will include lipid-polymer-targeting ligand. Additional polymer chains added to the lipid mixture at the time of liposome formation and in the form of a lipid-polymer conjugate result in polymer chains extending from both the inner and outer surfaces of the liposomal lipid bilayers. Addition of a lipid- polymer conjugate at the time of liposome formation is typically achieved by including between 0.5-20 mole percent of the polymer-derivatized lipid with the remaining liposome forming components, e.g., vesicle-forming lipids, Exemplary methods of preparing polymer-derivatized lipids and of forming polymer-coated liposomes have been described in U.S. Pat. Nos. 5,013,556, 5,631,018 and 5,395,619, which are incorporated herein by reference. It will be appreciated that the hydrophilic polymer may be stably coupled to the lipid, or coupled through an unstable linkage, which allows the coated liposomes to shred the coating of polymer chains as they circulate in the bloodstream or in response to a stimulus.

Alternatively, an antibody-lipid derivative may be first formed and then incorporated into a liposome. As an example, an antibody is coupled to the maleimide group of a free DSPE-PEG molecule. The antibody-coupled DSPE-PEG molecule is then employed to form vesicles.

After formation of the liposomes, a targeting ligand is incorporated to achieve a cell-targeted, therapeutic liposomes. The targeting ligand is incorporated by incubating the pre-formed liposomes with the lipid-polymer-ligand conjugate, prepared as described above. The pre-formed liposomes and the conjugate are incubated under conditions effective to association with the conjugate and the liposomes, which may include interaction of the conjugate with the outer liposome bilayer or insertion of the conjugate into the liposome bilayer. More specifically, the two components are incubated together under conditions which achieve associate of the conjugate with the liposomes in such a way that the targeting ligand is oriented outwardly from the liposome surface, and therefore available for interaction with its cognate receptor. It will be appreciated that the conditions effective to achieve such association or insertion are determined based on several variables, including, the desired rate of insertion, where a higher incubation temperature may achieve a faster rate of insertion, the temperature to which the ligand can be safely heated without affecting its activity, and to a lesser degree the phase transition temperature of the lipids and the lipid composition. It will also be appreciated that insertion can be varied by the presence of solvents, such as amphipathic solvents including polyethyleneglycol and ethanol, or detergents.

The targeting conjugate, in the form of a lipid-polymer-ligand conjugate, will typically form a solution of micelles when the conjugate is mixed with an aqueous solvent. The micellar solution of the conjugates is mixed with a suspension of pre-formed liposomes for incubation and association of the conjugate with the liposomes or insertion of the conjugate into the liposomal lipid bilayers. The incubation is effective to achieve associate or insertion of the lipid-polymer-antibody conjugate with the outer bilayer leaflet of the liposomes, to form an immunoliposome.

After preparation, the immunoliposomes preferably have a size of less than about 200 nm, preferably of between about 85-120 nm, and more preferably of between 90-110 nm, as measured, for example, by dynamic light scattering at 30[deg.] or 90[deg.].

Liposome compositions are typically prepared with lipid components present in a molar ratio of about 30-75 percent vesicle-forming lipids, 25-40 percent cholesterol, 0.5-20 percent polymer derivatized lipid, and 0.0001-10 mole percent of the lipid derivative employed for antibody coupling.

Generally, a therapeutic drug is incorporated into liposomes by adding the drug to the vesicle forming lipids prior to liposome formation, as described below, to entrap the drug in the formed liposome. If the drug is hydrophobic the drug is added directly to the hydrophobic mixture. If the drug is hydrophilic the drug can be added to the aqueous medium which covers the thin film of evaporated lipids.

The liposomes to be used in the present invention include an anti-tumor agent. Antitumor compounds contemplated for use in the invention include, but are not limited to, plant alkaloids, such as vincristine, vinblastine and etoposide; anthracycline antibiotics including doxorubicin, epirubicin, daunorubicin; fluorouracil; antibiotics including bleomycin, mitomycin, plicamycin, dactinomycin; topoisomerase inhibitors, such as camptothecin and its analogs; and platinum compounds, inducing cisplatin and its analogs, such as carboplatin. Other traditional chemotherapeutic agents suitable for use are known to those of skill in the art and includes, asparaginase, busulfan, chlorambucil, cyclophosphamide, cytarabine, dacarbazine, estramustine phosphate sodium, floxuridine, fluorouracil (5-FU), hydroxyurea (hydroxycarbamide), ifosfamide, lomustine (CCNU), mechlorethamine HCl (nitrogen mustard), melphalan, mercaptopurine, methotrexate (MTX), mitomycin, mitotane, mitoxantrone, procarbazine, streptozocin, thioguanine, thiotepa, amsacrine (m-AMSA), azacitidine, hexamethylmelamine (HMM), mitoguazone (methyl-GAG; methyl glyoxal bis-guanylhydrazone; MGBG), semustine (methyl-CCNU), teniposide (VM-26) and vindesine sulfate.

In one embodiment of the invention, the liposomes have a size suitable for extravasation into a solid tumor. This is particularly useful where the liposomes also includes a surface coating of a hydrophilic polymer chain to extend the blood circulation lifetime of the liposomes. Liposomes remaining in circulation for longer periods of time, e.g., more than about 2-5 hours, are capable of extravasating into tumors and sites of infection, which exhibit compromised leaky vasculature or endothelial barriers. Such liposomes are typically between about 40-200 nm, more preferably between 50-150 nm, most preferably between 70-120 nm.

The selected agent is incorporated into liposomes by standard methods, including (i) passive entrapment of a water-soluble compound by hydrating a lipid film with an aqueous solution of the agent, (ii) passive entrapment of a lipophilic compound by hydrating a lipid film containing the agent, and (iii) loading an ionizable drug against an inside/outside liposome pH gradient. Other methods, such as reverse-phase evaporation, are also suitable.

Alternatively, the drug may be incorporated into preformed liposomes by active transport mechanisms. Typically, in this case drug is taken up in liposomes in response to a potassium or hydrogen ion concentration differential (Mayer, 1986; Mayer 1989).

After liposome formation, the liposomes can be sized to obtain a population of liposomes having a substantially homogeneous size range, typically between about 0.01 to 0.5 microns, more preferably between 0.03-0.40 microns. One effective sizing method for REVs and MLVs involves extruding an aqueous suspension of the liposomes through a series of polycarbonate membranes having a selected uniform pore size in the range of 0.03 to 0.2 micron, typically 0.05, 0.08, 0.1, or 0.2 microns. The pore size of the membrane corresponds roughly to the largest sizes of liposomes produced by extrusion through that membrane, particularly where the preparation is extruded two or more times through the same membrane. Homogenization methods are also useful for down-sizing liposomes to sizes of 100 nm or less (24).

Liposomes carrying an entrapped agent and bearing surface-bound targeting ligands, i.e., targeted, therapeutic liposomes, may be prepared by any of these approaches. A preferred method of preparation is the insertion method, where pre-formed liposomes and are incubated with the targeting conjugate to achieve insertion of the targeting conjugate into the liposomal bilayers. In this approach, liposomes are prepared by a variety of techniques, such as those detailed in (23), and specific examples of liposomes prepared in support of the present invention will be described below. Typically, the liposomes are multilamellar vesicles (MLVs) or unilamellar resides (ULVs).

MLVs can be formed by simple lipid-film hydration techniques. In this procedure, a mixture of liposome-forming lipids of the type detailed above dissolved in a suitable organic solvent is evaporated in a vessel to form a thin film, which is then covered by an aqueous medium. The lipid film hydrates to form MLVs, typically with sizes between about 0.1 to 10 microns.

ULVs can be formed by the repeated freeze-thawing method. In this method 1-2-oleoyl-3-sn-glycerophosphocholine and Chol, or DSPC and Chol (molar ratio 3:2) is mixed with mPEGDSPE (0.5-5 mol% of phospholipid). Liposomes are subsequently extruded several times through polycarbonate filters with defined pore sizes of 0.1, 0.08 and 0.05 µm. This yields liposomes typically with sizes of 70-120 nm diameters. The size of the liposomes may be determined by dynamic light scattering. Liposome concentration can be measured using a standard phosphate assay.

The anti-EGFR immunoliposomes obtainable by any of the above described methods has clinical relevance and can be used in second and higher-line treatment of human patients suffering from cancer, particularly a cancer represented by a locally advanced or metastatic tumor. The immunoliposome contemplated for use in the present invention comprises an antibody or an antibody fragment, which recognizes and binds to an EGF receptor antigen on the surface of a solid tumor. In particular, the immunoliposome comprises a Fab, Fab', F(ab')₂, Fabc, Fv fragment, or is a single-chain antibody.

The immunoliposomes contemplated for use in the present invention further comprises an anti-tumor agent, particularly anti-tumor agent selected from the group consisting of doxorubicin, epirubicin and vinorelbine, particularly doxorubicine.

The immunoliposome according to the invention may be administered to a human patient in form of a pharmaceutical composition comprising said immunoliposome together with a pharmaceutically acceptable carrier and/or a diluent and/or an excipient. Formulation of the pharmaceutical composition according to the invention can be accomplished according to standard methodology known to those skilled in the art.

The immunoliposomes according to the invention or a pharmaceutical compositions comprising said immunoliposomes may be administered to a subject in the form of a solid, liquid or aerosol at a suitable, pharmaceutically effective dose. Examples of solid compositions includes pills, creams, and implantable dosage units. Pills may be administered orally. Therapeutic creams may be administered topically. Implantable dosage units may be administered locally, for example, at a tumor site, or may be implanted for systematic release of the therapeutic composition, for example, subcutaneously. Examples of liquid compositions include formulations adapted for infusions, formulations adapted for injection intramuscularly, subcutaneously, intravenously, intra-arterially, and formulations for topical and intraocular administration. Examples of aerosol formulations include inhaler formulations for administration to the lungs.

The immunoliposomes according to the invention or a pharmaceutical compositions comprising said immunoliposome may be administered by standard routes of administration. In general, the composition may be administered by topical, oral, rectal, nasal, interdermal, intraperitoneal, or parenteral (for example, intravenous, subcutaneous, or intramuscular) routes. In addition, the composition may be incorporated into sustained release matrices such as biodegradable polymers, the polymers being implanted in the vicinity of where delivery is desired, for example, at the site of a tumor. The method includes administration of a single dose, administration of repeated doses at predetermined time intervals, and sustained administration for a predetermined period of time.

It is well known to those skilled in the pertinent art that the dosage of a pharmaceutical composition will depend on various factors such as, for example, the condition of being treated, the particular composition used, and other clinical factors such as weight, size, sex and general health condition of the patient, body surface area, the particular compound or composition to be administered, other drugs being administered concurrently, and the route of administration.

The immunoliposome according to the invention or the composition comprising said immunoliposomes may be administered in combination with an biologically active substance or compound or other compositions comprising said biologically active substance or compound, particularly an anti-tumor compound, particularly at least one cytostatic compound, particularly a compound selected from the group consisting of particularly a compound selected from the group consisting of daunomycin, idarubicin, mitoxantrone, mitomycin, cisplatin and other Platinum analogs, vincristine, epirubicin, aclacinomycin, methotrexate, etoposide, doxorubicine, cytosine arabinoside, fluorouracil and other fluorinated pyrimidines, purines, or nucleosides, especially gemcitabine, bleomycin, mitomycin, plicamycin, daatinomyoin, cyclophosphamide and derivatives thereof, thiotepa, BCNU, paclitaxel , docetaxel and other taxane derivatives and isolates, camptothecins, polypeptides, a nuclei acid, a nucleic acid having a phosphorothioate internucleotide linkage, and a nucleic acid having a polyamide intemucleotide linkage, but especially doxorubicin, epirubicin and vinorelbine, together with an antibody according to the present invention and, optionally, a pharmaceutically acceptable carrier and/or a diluent and/or an excipient.

Pharmaceutically active matter, particularly the anti-tumor compounds which are entrapped in the immunoliposomes, may be present in amounts between 0.1 mg/m² and 2.5 g/m² of body surface and per dose. Generally, the regime of administration should be in the range of between 0.5 mg/m² and 1000 mg/m² of the anti-tumor compound according to the invention, particularly in a range of between 1.0 mg/m² to 500 mg/m², and particularly in a range of between 5.0 mg/m² and 250 mg/m², particularly in a range of between 10.0 mg/m² and 150 mg/m², with all individual numbers falling within these ranges also being part of the invention. If the administration occurs through continuous infusion a more proper dosage may be in the range of between 0.01 µg and 10 mg units per kilogram of body weight per hour with all individual numbers falling within these ranges also being part of the invention.

The antibody concentration of the immunoliposome is in a range of between 1 µg to 150 µg of antibody or antibody fragment per µmol phospholipid, particularly in a range of 5 µg to 100 µg of antibody or antibody fragment per µmol phospholipid, particularly in a range of 10 µg to 100 µg of antibody or antibody fragment per µmol phospholipid, particularly in a range of 20 µg to 50 µg of antibody or antibody fragment per µmol phospholipid, particularly in a range of 30 µg to 40 µg of antibody or antibody fragment per µmol phospholipid.

The immunoliposomal preparation of the present invention may be prepared in the form of a pharmaceutical composition containing the isolated and purified immunoliposome dissolved or dispersed in a pharmaceutically acceptable carrier well known to those skilled in the art, for parenteral administration by, e. g., intravenous, subcutaneous or intramuscular injection or by intravenous drip infusion.

As to a pharmaceutical composition for parenteral administration, any conventional additives may be used such as excipients, adjuvants, binders, disintegrants, dispersing agents, lubricants, diluents, absorption enhancers, buffering agents, surfactants, solubilizing agents, preservatives, emulsifiers, isotonizers, stabilizers, solubilizers for injection, pH adjusting agents, etc.

Acceptable carriers, diluents and adjuvants which facilitates processing of the active compounds into preparation which can be used pharmaceutically are non-toxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl orbenzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counterions such as sodium; metal complexes (e.g. Zn-protein complexes); and/or non-ionic surfactant such as TWEEN®, PLURONICS® or polyethylene glycol (PEG).

The form of administration of the pharmaceutical composition may be systemic or topical. For example, administration of such a composition may be various parenteral routes such as subcutaneous, intravenous, intradermal, intramuscular, intraperitoneal, intranasal, transdermal, buccal routes or via an implanted device, and may also be delivered by peristaltic means.

Administration will generally be parenterally, eg intravenously, particularly in form of an infusion. Preparations for parenteral administration include sterile aqueous or non-aqueous solutions, suspensions and emulsions. Non-aqueous solvents include without being limited to it, propylene glycol, polyethylene glycol, vegetable oil such as olive oil, and injectable organic esters such as ethyl oleate. Aqueous solvents may be chosen from the group consisting of water, alcohol/aqueous solutions, emulsions or suspensions including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose) and others. Preservatives may also be present such as, for example, antimicrobials, anti-oxidants, chelating agents, inert gases, etc.

The pharmaceutical composition may further comprise proteinaceous carriers such as, for example, serum albumine or immunoglobuline, particularly of human origan. Further biologically active agents may be present in the pharmaceutical composition of the invention dependent on its intended use.

In one aspect of the invention, immunoliposomes (ILs) as described herein were generated that bind VEGFR to provide efficient antibody-directed intracellular delivery of anticancer drugs into target cells to study whether it is possible by this approach to overcome drug resistance mechanisms, which remain an important obstacle towards better outcomes in cancer therapy.

ILs may be constructed modularly with various MAb or MAb fragments, including chimeric antibodies such as, for example, Fab' from DC101 covalently linked to stabilized liposomes containing various drugs or probes.

VEGFR-overexpressing cells, for example, such as in the Rip1Tag2, the PyMT or the HT-29 models, can be treated with the so-produced ILs. The injection of the anti-immunoliposomes loaded with 5 mg/kg doxorubicine induced a significant reduction of blood vessel density within a timeframe of 2-6 weeks. The effect was specific, since lymphatic endothelium, which expresses VEGFR3, was not affected by the therapy. In addition, cells that did not express VEGFR2 showed no relevant uptake of the immunoliposomes.

The response of the targeted blood vessels depended both on the localization and the morphology of the vessel. First, the effect was most prominent on vessels localized in the tumor, whereas vessels in healthy tissue were less vulnerable. This probably reflects the differential expression of the VEGFR2 in quiescent and tumor-associated vasculature. Secondly, capillaries consisting of a single layer of endothelial cells strongly responded to anti-VEGFR2-immunoliposomes resulting in vessel disruption and apoptosis of endothelial cells. Endothelial cells sitting on a layer of smooth muscle cells were less prone to apoptosis when treated with anti-VEGFR2-immunoliposomes.

Finally, the effect of the anti-angiogenic therapy with immunoliposomes led to a strong tumor regression. In the two transgenic mouse models, the efficacy of this therapy was significantly stronger than the effect of pegylated, but not targeted, liposomal doxorubicin. However, the difference was much smaller in the xenograft model. This is supposedly due to the fact that xenograft models are less well vascularized than transgenic tumor models.

The immunoliposomes according to the present invention and as dislosed herein thus provide efficient and targeted drug delivery to VEGFR-overexpressing tumor cells.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended claims, and all changes which come within the meaning and range of equivalency are intended to be embraced therein.

Various references are cited throughout this specification, each of which is incorporated herein by reference in its entirety.

The present invention is further described by reference to the following non-limiting figures and examples.

### The figures show:

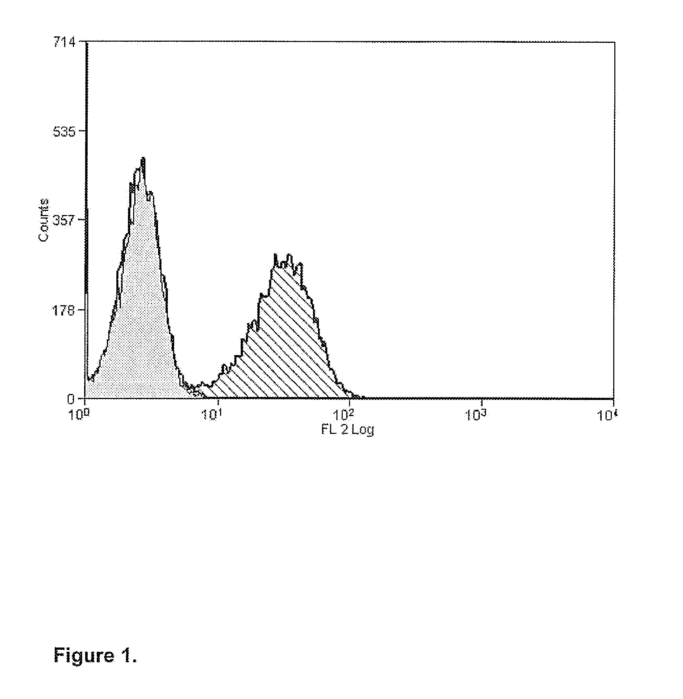

Figure 1 shows the uptake of VEGFR2-targeted immunoliposomes in MS-1 cells. The uptake of anti-VEGFR2 immunoliposomes was evaluated in target MS-1 cells by flow cytometric assay. Cells were incubated with DilC₁₈(3)-DS-labeled immunoliposomes containing DC101-Fab' or with control liposomes prepared identically as immunoliposomes except for omission of the MAb fragment. A shift of one-order-of-magnitude was detected for anti-VEGFR2 immunoliposomes in target cells compared to non-targeted liposomes.

Open histograms: MS-1 cells only without treatment. Filled histograms: control DilC₁₈(3)-DS-labefed liposomes. Dashed histograms: anti-VEGFR2 immunoliposomes.

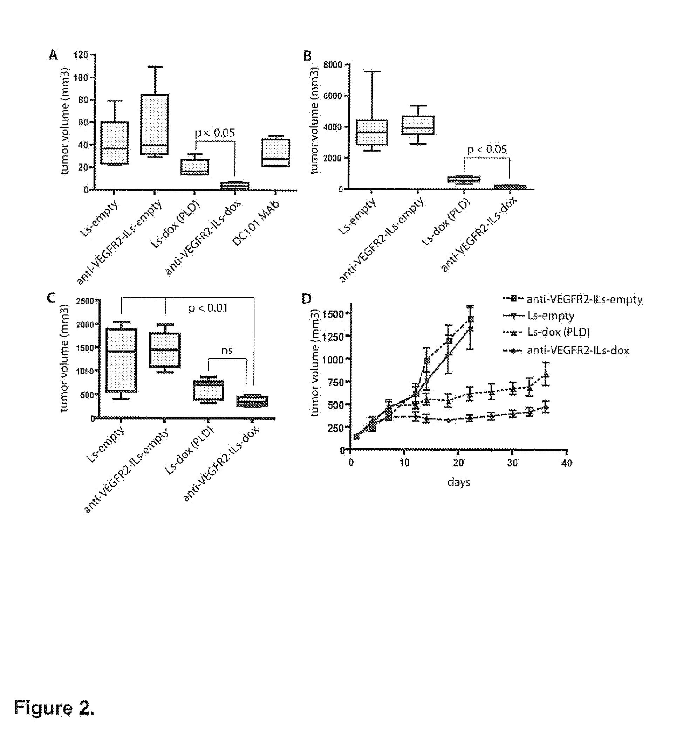

Figure 2 shows the effect of anti-VEGFR2-immunoliposomes on tumor growth. In the Rip1Tag2 and the PyMT models, anti-VEGFR2-immunoliposomes induce tumor regression which is significantly stronger than in the other groups. In the HT-29 xenograft model, anti-VEGFR2-immunoliposmes efficiently inhibit further tumor growth but the difference to the pegylated liposomal doxorubicine is not statistically significant (see main text). **(A)** Tumor volume in the Rip1Tag2 tumor mouse model, **(B)** tumor volume in the PyMT breast cancer mouse model, **(C)** tumor volume in the HT-29 xenograft model. The tumor volume in panels **A-C** was assessed after bi-weekly injections for a period of two weeks. The p value was calculated using the Kruskal-Wallis test, whiskers = interquartile range. **(D)** Kinetic of tumor growth in the HT-29 xenograft model. Tumor growth arrest is achieved first in the anti-VEGFR2-immunoliposomes group and about two days later in the PLD group. Thereafter, the curves of the anti-VEGFR2-immunoliposomes group and the PLD group run roughly parallel.

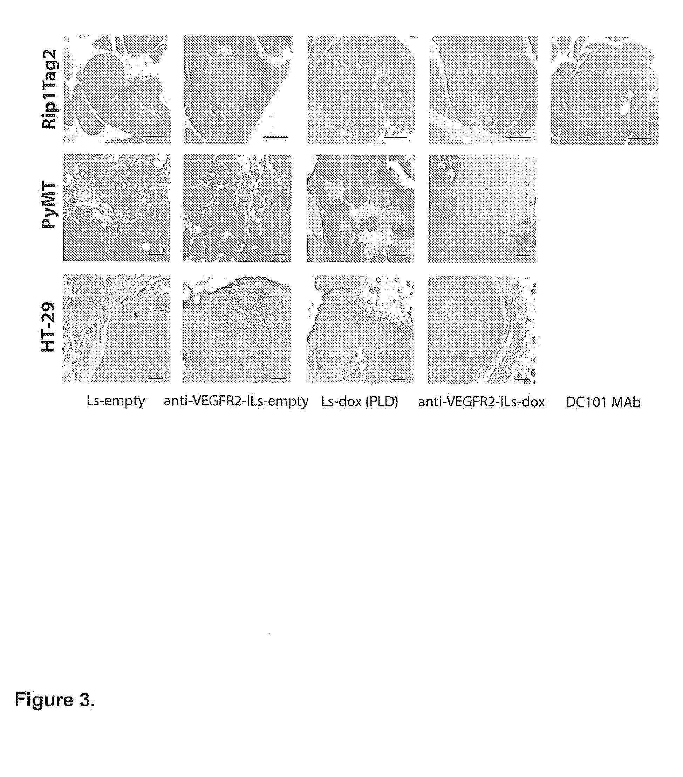

Figure 3 shows the H&E staining of tumor sections. Hemorrhagic lacunae are visible in the center of tumors treated with anti-VEGFR2-ILs-dox in the Rip1Tag2 and the PyMT tumor mouse model (top rows). In contrast, there are no hemorrhagic necroses in the HT-29 xenograft tumor sections (bottom row).Size bar = 200 µm.

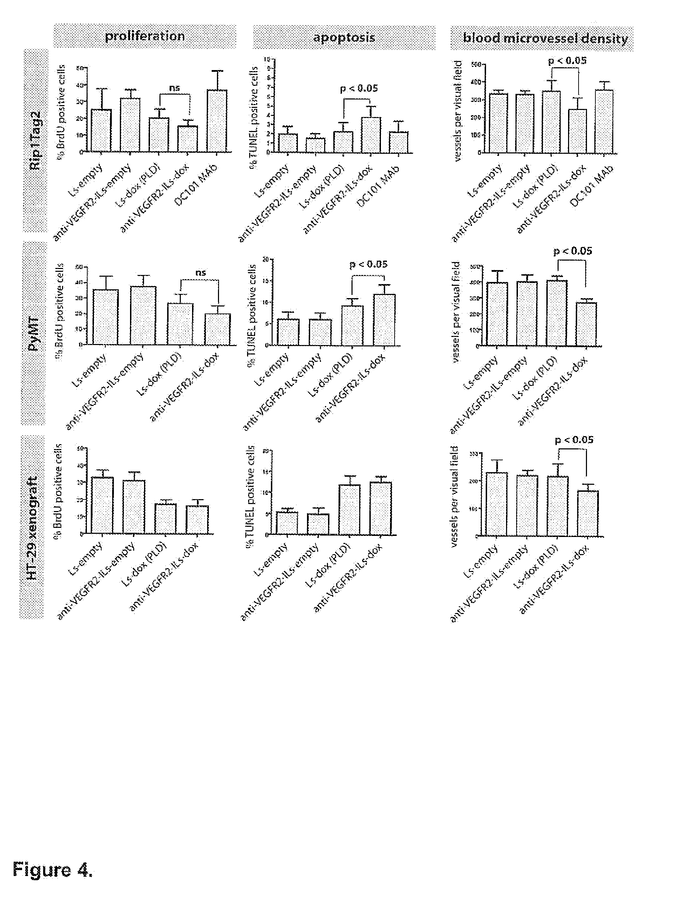

Figure 4 shows graphs representing proliferation, apoptosis and blood microvessel density in the Rip1Tag2 (top row), the PyMT (middle row) and the HT-29 xenograft model (bottom row). There is a trend for reduced proliferation in the anti-VEGFR2-ILs-dox group compared to the PLD group, but the difference is not statistically significant. Apoptosis is significantly increased in the anti-VEGFR2-ILs-dox group compared to all other cohorts. Blood microvessel density is significantly and specifically reduced in the anti-VEGFR2-ILs-dox cohorts in all three models.
Bars = mean ± standard deviation, p values were calculated using one-way anova testing with Newman-Keuls post test.

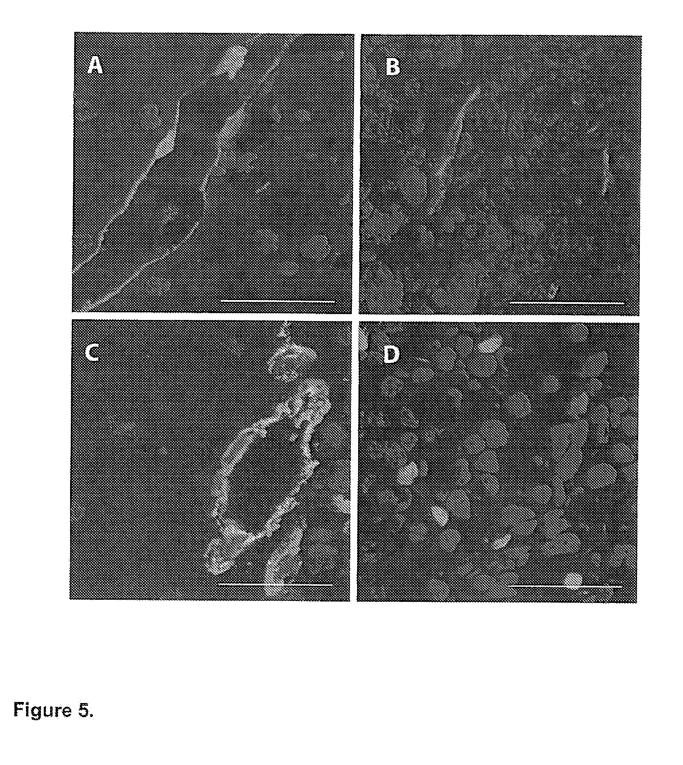

Figure 5 shows the immunofluorescence analysis of tumor-associated and physiological vessels in tumor sections. (A) Vessel in the exocrine pancreas of a Rip1Tag2 mouse treated with anti-VEGFR2-ILs-dox. This is an intact vessel formed of a single layer of endothelial cells. Green = staining for CD31, blue = DAPI. (B) Capillaries in the tumors of a Rip1Tag2 mouse treated with PLD. The endothelial cells form again a single layer tube. Green = staining for CD31, blue = DAPI. (C) Vessel at the rim of a tumor in a Rip1Tag2 mouse treated with anti-VEGFR2-ILs-dox. This is a larger vessel composed of an endothelial and a smooth muscle cell layer. The vessel stayed intact despite the treatment with anti-VEGFR2-ILs-dox. Green = CD31, red = smooth muscle, blue = DAPI. (D) Apoptotic endothelial cells in a tumor of a Rip1Tag2 mouse treated with anti-VEGFR2-ILs. The endothelial cells still express CD31 but they have become pyknotic and do not form capillaries any more. Green = CD 31, blue = DAPI. Size bar A-D = 10 µm.

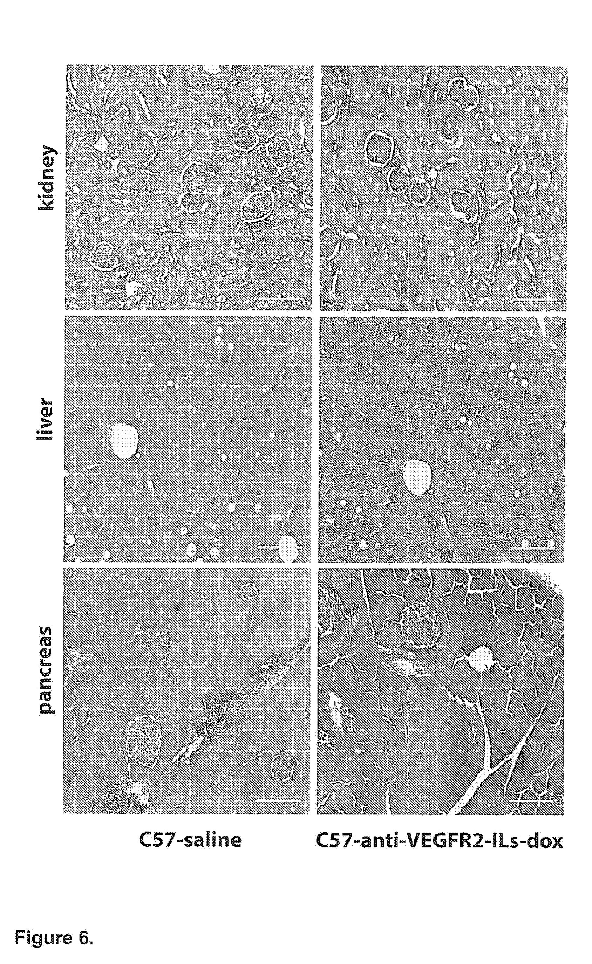

Figure 6 shows the H&E stained sections of C56/BI6 mice injected with anti-VEGR2-ILs-dox or saline, as indicated. The mice were sacrificed 18 months after treatment and the interval organs were analysed morphologically for signs of toxicity. There was no morphological difference between organs in the saline cohort and in the anti-VEGFR2-ILs-dox cohort. Size bar = 100 µm.

The foregoing description will be more fully understood with reference to the following Examples. Such Examples, are, however, exemplary of methods of practising the present invention and are not intended to limit the scope of the invention.

The following Examples illustrate the invention:

### Example 1: Materials and Methods.

Reagents for liposome preparation included: DilC₁₈(3)-DS (Molecular Probes; Leiden, Netherlands); DSPC, cholesterol, and mPEG-DSPE (Avanti Polar Lipids; Alabaster, AL, USA); Mal-PEG(2000/3400)-DSPE (Nektar; Huntsville, AL, USA); organic solvents, and other chemicals of reagent purity (Sigma-Aldrich AG; Buchs, Switzerland). Doxorubicin (Adriblastin RD®; Pfizer AG, Zürich, Switzerland) and pegylated liposomal doxorubicin (Caelyx®, Essex Chemie AG, Luzern, Switzerland) were obtained commercially from the pharmacy. MAb DC101 was kindly provided by ImClone Systems Inc., New York, USA.

### Liposome preparation

Liposomes were prepared by a lipid film hydration-extrusion method using repeated freeze-thawing to hydrate the lipid films (Szoka, 1980 No.52). Liposome were composed of DSPC and cholesterol (molar ratio 3:2) with mPEG-DSPE (0.5∼5 mol% of phospholipid). Following hydration, liposomes were subsequently extruded 10 times through polycarbonate filters with defined pore sizes of 0.1 µm, followed by 7 times extrusion through filters with 0.08 µm pore size. Liposome size was determined by dynamic light scattering (typically 80-100 nm). The phospholipid concentration in the liposome solution was measured utilizing a standard phosphate assay (Bartlett, 1959 No.39), typically after purification on a G-75 gel filtration column.

For uptake and internalization studies, liposome were labelled with 0.1-0.3 mol% DilC₁₈(3)-DS, a fluorescent lipid that can be stably incorporated into liposomal membranes (Claassen, 1992; Litzinger et al., 1994).

For encapsulation of doxorubicine, the remote-loading method using ammonium sulfate was performed (Haran et al., 1993; Lasic et al., 1992). First, dry lipids were rehydrated in 250 mM ammonium sulfate at pH 5.5, followed by extrusion as described above. Free ammonium sulfate was removed by size-exclusion chromatography using a Sephadex G-75 column/HEPES buffered saline (pH 7.0). Liposomes were then incubated with doxorubicin for 30 min at 60 °C. Under these conditions, loading efficiencies were typically in the range of 95-100 % when 150 µg drug per µmol phospholipid was used. All unencapsulated doxorubicin was removed by size-exclusion chromatography using a Sephadex G-75 column. In addition, pegylated liposomal doxorubicin (PLD/Caelyx®/Doxil®) was obtained commercially.

### Preparation of MAb fragments and immunoliposomes

DC101 is a rat MAb against mouse VEGFR-2. For preparation of DC101-Fab', intact DC101 MAb was cleaved and reduced as previously described for anti-EGFR MAb C225 (cetuximab) (Mamot, 2003 No.65). Briefly, after cleavage of the Fc region with pepsin, the resulting DC101-F(ab)₂ was reduced with 2-mercaptoethylamine under argon for 15 min at 37 °C, and then recovered by gel filtration using Sephadex G-25. The efficiency of cysteine reduction was assayed using Ellmann's reagent, and was typically greater than or around 90 %.

For immunoliposomes, Fab' were covalently conjugated to maleimide groups at the termini of PEG-DSPE chains (Mal-PEG-DSPE) (Nellis, 2005 No.96). Conjugation was via a thioether linkage between the free thiol near the COOH terminus of the MAb fragment and terminal maleimide groups of the PEG-DSPE chains. Conjugation efficiencies were typically 50-60 % for DC101-Fab'. For incorporation into preformed liposomes or commercial pegylated liposomal doxorubicin (PLD), MAb fragment conjugates (Fab'-Mal-PEG-DSPE) which form micellar solutions, were incorporated into liposomesby coincubation at 55 °C for 30 min at protein/liposome ratio of 60 µg Fab'/µmol PL. As a result, the conjugates become inserted in the outer lipid layer of the liposomes via hydrophobic DSPE domains (Ishida, 1999 No.94; Nielsen, 2002 No.54). Unincorporated conjugates and free drug were separated from immunoliposomes by Sepharose CL-4B gel filtration. The incorporation efficiency of conjugated MAb fragments was estimated by SDS-PAGE using a series of protein standards by comparing coomassie blue stained bands to standard Fab' preparation using densitometry (Mamot, 2003 No. 65). Typically, 50-60 % of added MAb conjugate was incorporated into immunoliposomes.

### Specific binding of anti-VEGFR2 immunoliposomes

For flow cytometry studies, 150,000 MS-1 or MDA-MB-231 cells were co-incubated in 12-well plates with saline (control), liposomes or VEGFR2-targeted immunoliposomes labeled with DilC₁₈(3)-DS for 2 h at 37 °C, washed extensively with PBS, followed by detaching and storing on ice until subjected to flow cytometry.

### Mice

Phenotypic and genotypic analyses of Rip1Tag2 and PyMT transgenic mice in a G57BI/6J background have been described previously. Wild-type C57BI/6J mice were used for organ toxicity studies. Athymic nude mice were hosts for the HT-29 xenograft experiments. Animals were maintained and treated in compliance with the guidelines of the Swiss Veterinary Office and the Cantonal Veterinary Office Basel-Stadt.

For therapeutic studies, mice were injected into the tail vein with 5 mg doxorubicine/kg anti-VEGFR2-ILs-dox, 5 mg doxorubicine/kg pegylated liposomal doxorubicine (PLD) or the same quantity of empty (immuno)liposomes and empty immunoliposomes, respectively, i.v. at day 0, 3, 7, and 10. In the Rip1Tag2 mouse model, we injected an additional cohort of mice with free DC101 at the same concentration as was present in the anti-VEGFR2-ILs-dox cohort (membrane-bound DC101). Tumor size was assessed after sacrifice using a caliper or a grid (for small tumors). The mass was calculated assuming an ellipsoid shape of the tumor.

For the xenograft experiments, 10⁶ HT-29 colon cancer cells in 100µl saline were injected subcutaneously in athymic mice. 4 weeks after engraftment treatment was started following the same protocol as for the transgenic tumor mouse models. Tumor size was assessed twice weekly for 6 weeks using a caliper, and the tumor size was calculated assuming an ellipsoid form of the tumor.

### Histologic analysis

Tissue was fixed in 4% paraformaldehyde overnight at 4°C and embedded in paraffin. For frozen sections, tissue was immersed overnight in 20% sucrose in NBS-Ca²⁺ (HEPES-buffered saline with 1 mmol/L CaCl₂), and embedded and snap-frozen in optimum cutting temperature compound (Tissue-Tek). Immunohistochemistry on paraffin and frozen sections was performed by quenching the slides in 3% H₂O₂ in PBS for 10 min, blocking in 5% goat serum for 1 h, incubation with primary antibody overnight at 4°C, incubation with secondary antibody for 30 min, and development with the ABC-kit of Vectastain (Vector Laboratories) and Sigma Fast 3.3'Diaminobenzidine tetrahydrochloride with metal enhancer (Sigma) or AEC-kit (Vector Laboratories). Blood and lymphatic vessels were visualized using an anti-CD31 or anti-Lyve1 primary antibody (Sigma), respectively.

For immunofluorescence stainings, frozen sections were permeabilized in 0.2% Triton X-100 in PBS, blocked in 5% goat serum for 1 h, and incubated with rat anti-CD31, anti-NG2 or anti-smo 1:100 (Sigma) as primary antibody for 1 h and with a fluorescent secondary antibody for 30 min.

### Proliferation and apoptosis assay

For bromodeoxyuridine (BrdU) staining (proliferation assay), BrdU [5 mg/mL in 10 mmol/L Tris-HCl (pH 7.4); 0.8% NaCl; 0.25 mmol/L EDTA] was injected at 100 µg BrdU per gram body weight i.p. 2 h before sacrifice. After antigen retrieval (2 N HCl for 1 h and trypsinization), pancreatic sections were incubated in a 1:100 solution of biotinylated mouse anti-BrdU antibody (Zymed). Incorporated BrdU was visualized with the ABC-kit of Vectastain (Vector Laboratories) and Sigma Fast 3.3'Diaminobenzidine tetrahydrochloride with metal enhancer (Sigma). For terminal deoxyribonucleotide transferase-mediated nick-end labeling (TUNEL; apoptosis) assays, antigen retrieval on slides was done by digestion with proteinase K (Fluka). Slides were incubated for 1 h in TUNEL reagent (Roche) at 37°C and apoptotic cells were visualized with the AEC-kit (Vector Laboratories).

### Microscopy

Immunohistochemical stainings were analyzed on a AxioVert microscope (Zeiss). Immunofluorescence staining were analyzed on a LSM510 META confocal microscope using LSM510 software (Zeiss). Histologic grading of islet and mammary tumors was done following the WHO International Classification of Rodent Tumors, Part II, The Mouse.

### Statistical analysis

Statistical analysis was performed using the GraphPad Prism software (GraphPad Software, Inc.). Tumor volume and mass were compared using nonparametric statistical analysis (Kruskal-Wallis test) with Dunn's post test. Proliferation, apoptosis, and blood and lymph vessel density were analyzed by parametric testing (one-way ANOVA and Newman-Keuls post test).

### Example 2: Construction of anti-VEGFR2 immunoliposomes derived from DC101.

Anti-VEGFR2 immunoliposomes were prepared by conjugation of 80-120 nm liposomes, (PC/Chol/PEG-PE) with MAb fragments directed against VEGFR2. Immunoliposomes contained Fab' derived from DC101, which is a monoclonal rat antibody binding mouse VEGFR2, not cross-reactive with the human analogue (Witte et al., Cancer and Metastasis Reviews 1998). DC101 binds to the extracellular domain (ECD) of VEGFR2 and thereby blocks activation by ligands such as VEGF.

Antibody preparation was checked by SDS-page gels, demonstrating the different steps of antibody modification. Routinely, the conjugates were purified before incorporation into preformed liposomes. Total yields achieved 30-40% for DC101 of initial protein.

### Example 3: Uptake of VEGFR2-targeted immunoliposomes in VEGFR2-overexpressing cells.

Binding and uptake of VEGFR2-targeted immunoliposomes in VEGFR2-overexpressing MS-1 cells was evaluated by flow cytometry. In this assay, liposomes were fluorescently labelled with DiIC₁₈(3)-DS with/without conjugated MAb fragments, and incubated with target MS-1 or control MDA-MB-231 cells for 2 h at 37 °C. immunoliposomes (dashed graph) containing DC101-Fab' showed an approximately 1 order-of-magnitude greater accumulation in VEGFR2-overexpressing MS-1 cells than did control liposomes (filled graph), which produced only background levels of fluorescence in these cells (Figure 1). In comparison, only minimal uptake in control MDA-MB-231 cells lacking the VEGF2 receptor was detected that was indistinguishable from that of non-targeted liposomes (data not shown). This result indicated selectivity for immunoliposome uptake between the two cell lines differing in VEGFR2 expression.

As reported before, the observation of minimal fluorescence uptake in target cells after incubation with control liposomes is consistent with the non-reactive properties of pegylated liposomes (Kirpotin et al., 1997b; Park et al., 1995), and also confirms that DiIC₁₈(3)-DS can be used as a stable liposome-based marker without significant exchange into cell membranes.

### Example 4: Effect of anti-VEGFR-2 immunoliposomes on tumor volume and tumor growth.

Cohorts of Rip1Tag2 (n=6 each), MMTV-neu (n=9 each) and nude mice xenografted with HT29 colon cancer cells (n=8 each) were injected with 5 mg doxorubicine/kg anti-VEGF-R2 immunoliposomes i.v. at day 0, 3, 7, 10. Control groups for the Rip1Tag2 mice included non-targeted empty liposomes (4 injections i.v. with the same concentration as for the interventional group), targeted empty immunoliposomes (4 injections analogous to the interventional group), non-targeted liposomes containing doxorubicine (again in analogy to the interventional group), and the antibody alone (4 injections i.v. using the same concentration of the antibody as used in anti-VEGFR2 ILs). For MMTV-neu mice and xenografted nude mice, controls consisted of targeted empty immunoliposomes, empty liposomes and untargeted doxorubicine-loaded liposomes. Rip1Tag2 and MMTV-neu mice were sacrificed at day 15 and the tumor volume was determined. The size of the xenografted tumors in nude mice was assessed twice weekly for a period of 5 weeks.

In the Rip1Tag2 mouse model, tumor volume in the interventional group was significantly reduced compared to all controls groups (non-parametrical analysis using Dunn's post test, p<0.05, Figure 2A). The tumor volume in the interventional group was 3.58±2.4 mm³, as compared to 16.5±6.9 mm³ in the non-targeted liposomal doxorubicine (PLD) group, 36.8±20.6 mm³ in the non-targeted empty liposomes group, 39.8±30.0 mm³ in the targeted empty immunoliposomes group and 28.0±11.6 mm³ in the group with the antibody alone (values = median ± standard deviation), In particular, immunoliposomes targeted against tumor vasculature were superior to liposomal (untargeted) doxorubicine, underlining the power of targeting tumor vasculature instead of the tumor cells themselves.

In the PyMT-neu mouse, the median tumor volume in the interventional group was 167±74.3 mm³, as compared to 611±172 mm³ in the liposomal doxorubicine group, 3951±1544 mm³ in the empty liposomes group and 4080±741 mm³ in the targeted empty immunoliposomes group. Again, the tumor volume in the targeted doxorubicine group was significantly smaller than in all included control groups (p<0.05, Dunn's post-test, Figure 2B),

In nude mice carrying xenografted HT29 colon carcinoma cells, the tumor growth was effectively inhibited in both the group injected with liposomal doxorubicine and in the group with targeted, doxorubicine-loaded immunoliposomes (Figure 2C). However, the onset of the growth inhibition occurred earlier in the mice treated with targeted doxorubicine than in mice injected with untargeted liposomal doxorubicine. In a multivariate rank analysis, the targeted and the untargeted doxorubicine group were not significantly different from one another (p > 0.05). However, there was a clear trend towards lower tumor volume in the anti-VEGR2-immunoliposomes group (Figure 2C). The two groups with empty (immuno-)liposomes had a significantly higher tumor load than the groups with doxorubicine-containing (immuno-)liposomes (p<0.001).

Thus, in both transgenic tumor models, targeted doxorubicine-loaded immunoliposomes were more efficient in terms of growth suppression than non-targeted liposomal doxorubicine. In the xenograft model, there was a trend towards higher efficiency of anti-VEGFR2-immunoliposomes, but the difference was not statistically significant. Analysis of the tumors by H&E staining revealed large hemorrhagic necrosis in tumors treated with anti-VEGFR2-immunoliposomes (Figure 3). Again, the effect was more pronounced in the transgenic models as compared to the xenograft model. Of note, because of the central hemorrhagic necrosis in tumors treated with anti-VEGFR2-immunoliposomes, there is an inherent tendency to overestimate the actual tumor volume in this interventional group.

### Example 5: Effect of anti-VEGFR-2 immunoliposomes on tumor cell proliferation, apoptosis and microvessel density.

In each of the three aforementioned experimental systems, we analysed tumor cell proliferation and apoptosis, using a BrdU and TUNEL-based approach, respectively (Figure 4, first two columns).

In the Rip1Tag2 model, tumor cell proliferation was significantly reduced in the targeted doxorubicine-loaded immunoliposomes group, as compared to the targeted and untargeted empty liposomes groups and the antibody alone (p < 0.05, Newman-Keuls post-test). The relative reduction of tumor cell proliferation in mice treated with targeted doxorubicine was 24% as compared to mice injected with liposomal doxorubicine, but this difference was not statistically significant (Figure 4, first column, p>0.05, Newman-Keuls post test).

In contrast, apoptosis was significantly increased in mice treated with anti-VEGFR2-immunoliposomes as compared to all the control groups (p<0.05, Newman-Keuls post test). In particular, the relative increase of apoptotic cells in the anti-VEGFR2-immunoliposomes group was 72% if compared with the pegylated liposomal doxorubicine group.

The same effect could be observed in the PyMT breast cancer model. Again, BrdU staining indicates a reduced proliferation of tumor cells in mice treated with anti-VEGFR2-immunoliposomes, but the difference is not significant if compared with the PLD-group. However, apoptosis is significantly increased by 30% in tumors treated with anti-VEGFR2-immunoliposomes instead of PLD (Figure 4, second column, p < 0.05, Newman-Keuls post test).

In the HT-29 xenograft model, at the time of analysis (day 42, 4 weeks after termination of the therapy), there was no difference in proliferation or apoptosis when comparing the anti-VEGFR2-immunoliposomes group with the PLD group (Figure 4, first and second column).

We next determined both blood and lymphatic microvessel density for all groups in the Rip1Tag2, the PyMT and the HT-29 xenograft model (Figure 4, last column; data for lymphatic vessels not shown). In the Rip1Tag2 mouse, the blood vessel density was reduced by 25-31 % in the anti-VEGFR2-immunoliposomes group compared to the other groups. This difference was statistically significant (p < 0.05, Newman-Keuls post test). In the PyMT breast cancer mouse, blood vessel density in the anti-VEGFR2-immunoliposomes group was reduced by 31-33% (p < 0.05, Newman-Keuls post test). Finally, in the HT-29 xenograft model, blood vessel density was reduced by 24-28% (p < 0.05, Newman-Keuls post test).

If the same analysis was performed for lymphatic vessels, there was no significant difference between the cohorts in all three models.

### Example 6: Disruption of small blood vessels by anti-VEGFR2-immunollposomes.

The injection of anti-VEGFR2-immunoliposomes induces a rapid decline of tumor-associated microvessels. However, when staining for CD31 positive vessels, a part of the vasculature is still present after treatment of tumors with anti-VEGFR2-immunoliposomes. To investigate the mechanisms of vessel disruption and to identify factors that protect vessels from the action of anti-VEGFR2-immunoliposomes or make them more vulnerable to treatment, the vessel morphology was analysed by immunofluorescence using antibodies directed against the endothelial cells themselves (anti-CD31), or against other components of the vessel wall such as pericytes (anti-NG2) or smooth muscle cells (anti-smo, Figure 5).

We found that single-layer endothelial cells forming capillaries in the exocrine pancreas of mice treated with anti-VEGFR2-immunoliposomes were still present (Figure 5A). Morphologically normal capillaries were present in mice treated with PDL (Figure 5B), whereas the number of intact capillaries was reduced by more than two-thirds in the tumors of mice treated with anti-VEGFR2-immunoliposomes (Figure 5D). Instead, DAPI staining shows pyknotic nuclei in the center of single interspersed cells which still express CD31 (Figure 5D). However, even in the tumors of mice injected with anti-VEGFR2-immunoliposomes we could still detect small vessels formed by endothelial cells sitting on a layer of smooth muscle cells (Figure 5C). No pericyte coverage of vessels was detected in tumors of control mice and mice treated with anti-VEGFR2-immunoliposomes.

Taken together, targeting of endothelial cells with anti-VEGFR2-immunoliposomes led to the disruption of microvessels and apoptosis of endothelial cells in the tumor. Capillaries in the exocrine pancreas were not affected by anti-VEGFR2 immunoliposomes, probably reflecting the low level of VEGFR2 expression in non-activated endothelial cells as compared to tumor-associated endothelium expressing a high quantity of VEGFR2. Endothelial cells sitting on a layer of smooth muscle cells appeared to benefit from a certain protection against the effect of anti-VEGFR2-immunoliposomes, since this type of vessels could still be detected after the treatment of mice.

### Example 7: Toxicity of anti-VEGFR2-immunoliposomes.

To evaluate the toxicity of anti-VEGFR2-immunoliposomes in comparison to pegylated liposomal doxorubicine, we injected cohorts of C57/BL6 mice (n=3) either with anti-VEGFR2-immunoliposomes, PLD, or empty immunoliposomes. All animals survived for 18 months without apparent signs of disease or distress. Histological analysis of the pancreas, the liver and the kidneys after sacrifice showed no morphological difference between treated mice and healthy controls (Figure 6). Also, lungs, intestine, spleen and bone marrow were unaltered.

We therefore conclude that the therapy with anti-VEGFR2-immunoliposomes was well tolerated and had no significant long-term toxicity.

## Claims

1. An immunoliposome comprising an antibody or an antibody fragment, which recognizes and binds to an VEGF receptor (VEGFR) antigen on the surface of a tumor cell or a tumor-asscociated endothelial cell and further encapsulating in the liposome an anti-tumor compound, for treatment of cancer, particularly a cancer represented by a locally advanced or metastatic tumor, in an individual, particularly animal or human.

2. An immunoliposome according to claim 1, wherein the VEGFR antigen is selected from a group consisting of VEGFR1, VEGFR2 or VEGFR3 antigens or combinations thereof.

3. An immunoliposome according to claim 2, wherein the VEGFR antigen is a VEGFR2 antigen.

4. An immunoliposome according to anyone of claims 1 to 3, wherein said endothelial cells are cells lining new vessels that are being formed in a growing tumor.

5. An immunoliposome according to any of the preceding claims, wherein the liposome encapsulates a cytostatic compound.

6. An immunoliposome according to any of the preceding clams, wherein the anti-tumor compound is a compound selected from the group consisting of daunomycin, idarubicin, mitoxantrone, mitomycin, cisplatin and other Platinum analogs, vincristine, epirubicin, aclacinomycin, methotrexate, etoposide, doxorubicin, cytosine arabinoside, fluorouracil and other fluorinated pyrimidines, purines, or nucleosides, especially gemcitabine, bleomycin, mitomycin, plicamycin, dactinomycin, cyclophosphamide and derivatives thereof, thiotepa, BCNU, paclitaxel, vinorelbine, docetaxel and other taxane derivatives and isolates, camptothecins, polypeptides, a nucleic acid, a nucleic acid having a phosphorothioate internucleotide linkage, and a nucleic acid having a polyamide internucleotide linkage.

7. An immunoliposome according to claims 6, wherein the cytotoxic compound is a compound selected from the group consisting of doxorubicin, epirubicin and vinorelbine.

8. An immunoliposome according to any of the preceding claims, wherein the tumor is still progressing.

9. An immunoliposome according to any of the preceding claims, wherein the treatment leads to a regression of the disease.

10. An immunoliposome according to any of the preceding claims, wherein the treatment shows no or substantially no toxic side effects.

11. An immunoliposome according to any of the preceding claims, wherein the treatment does not show substantial toxicity.

12. An immunoliposome according to any of the preceding claims, wherein the treatment shows no or substantially no toxic side effects at a concentration of between 5 mg/m² and 100 mg/m².

13. An immunoliposome according to any of the preceding claims, wherein the treatment shows no or substantially no toxic side effects at a concentration of up to 40 mg/m².

14. An immunoliposome according to any of the preceding claims, wherein the antibody or antibody fragment is covalently bound to the liposome membrane.

15. An immunoliposome according to any of the preceding claims, wherein the antibody is covalently conjugated to the terminus of a linker molecule anchored to the liposome.

16. An immunoliposome according to the previous claim, wherein the linker molecule is a polyethylene glycol.

17. An immunoliposome according to any of the preceding claims, wherein the antibody is a monoclonal antibody directed to the ligand-binding extracellular domain of the VEGF receptor.

18. An immunoliposome according to any of the preceding claims for the treatment of a cancer in an individual, particularly animal or human, selected from the group consisting of Kaposi's sarcoma, recurrent ovarian cancer, breast cancer, soft tissue sarcoma, glioma, melanoma, mesothelioma, transitional cell carcinoma of the urothelial tract, endometrial, pancreatic, small-cell and non-small-cell lung, hepatocellular, renal cell, esophageal, colorectal, anal, vaginal, vulvar, prostate, basal cell carcinoma of the skin head and neck, and cholangio carcinoma, which cancer is particularly represented by a locally advanced or metastatic tumor. particularly a tumor comprising tumor cells exhibiting VEGF receptor (VEGFR) antigen on the cell surface or, particularly, a tumor comprising tumor-asscociated endothelial cells, but especially endothelial cells lining new vessels that are being formed in a growing tumor and exhibiting VEGF receptor (VEGFR) antigen on the cell surface.

19. An immunoliposome according to any of the preceding claims for the treatment of a cancer in an individual, particularly animal or human, selected from the group consisting of prostate, pancreatic, kidney, urothelial, oesophageal, head and neck, colorectal, a hepatocellular cancer and a mesothelioma, which cancer is particularly represented by locally advanced or metastatic tumor, particularly a tumor comprising tumor cells exhibiting VEGF receptor (VEGFR) antigen on the cell surface or, particularly, a tumor comprising tumor-asscociated endothelial cells, but especially endothelial cells lining new vessels that are being formed in a growing tumor and exhibiting VEGF receptor (VEGFR) antigen on the cell surface.

20. An immunoliposome according to any of the preceding claims for use in the treatment of an individual, particularly, animal or human, who has cancer, particularly a cancer represented by a locally advanced or metastatic tumor, particularly a tumor comprising tumor cells exhibiting VEGF reeeptor (VEGFR) antigen on the cell surface or, particularly, a tumor comprising tumor-asscociated endothelial cells, but especially endothelial cells lining new vessels that are being formed in a growing tumor and exhibiting VEGF receptor (VEGFR) antigen on the cell surface.

21. Use of an immunoliposome according to any of the preceding claims for the preparation of a pharmaceutical composition for the treatment of cancer, particularly a cancer represented by a locally advanced or metastatic tumor, particularly a tumor comprising tumor cells exhibiting VEGF receptor (VEGFR) antigen on the cell surface or, particularly, a tumor comprising tumor-asscociated endothelial cells, but especially endothelial cells lining new vessels that are being formed in a growing tumor and exhibiting VEGF receptor (VEGFR) antigen on the cell surface.

22. A method of using an immunoliposome or a pharmaceutical composition according to any of the preceding claims for the preparation of a pharmaceutical for use in the treatment of an individual, particularly animal or human, who has cancer, particularly a cancer represented by a locally advanced or metastatic tumor, particularly a tumor comprising tumor cells exhibiting VEGF receptor (VEGFR) antigen on the cell surface or, particularly, a tumor comprising tumor-asscociated endothelial cells, but especially endothelial cells lining new vessels that are being formed in a growing tumor and exhibiting VEGF receptor (VEGFR) antigen on the cell surface.

23. A pharmaceutical composition comprising an immunoliposome according to any of the preceding claims together with a pharmaceutically acceptable carrier or excipient or a diluent for the treatment of cancer represented by a locally advanced or metastatic tumor, particularly a tumor comprising tumor cells exhibiting VEGF receptor (VEGFR) antigen on the cell surface or, particularly, a tumor comprising tumor-asscociated endothelial cells, but especially endothelial cells lining new vessels that are being formed in a growing tumor and exhibiting VEGF receptor (VEGFR) antigen on the cell surface.in an individual, particularly animal or human.

24. A pharmaceutical composition according to claim 23 for the treatment of breast cancer.

25. A pharmaceutical composition according to claim 23 for the treatment of a colorectal cancer.

26. A pharmaceutical composition comprising an immunoliposome according to any of the preceding claims, wherein said immunoliposomes encapsulates doxorubicin and further comprises antibody DC101 MAb or a fragment thereof, which still exhibits the specific binding properties of said antibody.

27. Pharmaceutical composition according to claims 23 to 26 for use in the treatment of an individual, particularly animal or human who has cancer, particularly a cancer represented by a locally advanced or metastatic tumor, particularly a tumor comprising tumor cells exhibiting VEGF receptor (VEGFR) antigen on the cell surface or, particularly, a tumor comprising tumor-asscociated endothelial cells, but especially endothelial cells lining new vessels that are being formed in a growing tumor and exhibiting VEGF receptor (VEGFR) antigen on the cell surface.

28. Use of the pharmaceutical composition according to any of the preceding claim for the preparation of a pharmaceutical composition for the treatment of cancer, particularly, a cancer represented by a locally advanced or metastatic tumor, particularly a tumor comprising tumor cells exhibiting VEGF receptor (VEGFR) antigen on the cell surface or, particularly, a tumor comprising tumor-asscociated endothelial cells, but especially endothelial cells lining new vessels that are being formed in a growing tumor and exhibiting VEGF receptor (VEGFR) antigen on the cell surface.

29. Method of treatment of an individual, particularly animal or human, who has cancer, particularly a cancer represented by a locally advanced or metastatic tumor, particularly a tumor comprising tumor cells exhibiting VEGF receptor (VEGFR) antigen on the cell surface or, particularly, a tumor comprising tumor-asscociated endothelial cells, but especially endothelial cells lining new vessels that are being formed in a growing tumor and exhibiting VEGF receptor (VEGFR) antigen on the cell surface, wherein
(a) the immunoliposome or the pharmaceutical composition of any of the preceding claims is administered to an individual, particularly animal or human; and
(b) the effect of the treatment is monitored based on one or more parameters selected from the group consisting of tumor volume, tumor growth, tumor cell proliferation, number of apoptotic cells and microvessel density.

30. Method for disruption of small blood vessels comprising tumor cells or tumor-associated endothelial cells, wherein
(a) the immunoliposome or the pharmaceutical composition of any of the preceding claims is administered to an individual, particularly animal or human; and
(b) the disruption of said blood vessels is monitored by immunofluorescence staining of the target cells.
